# EUROPEAN PATENT APPLICATION

(11) **EP 2 833 313 A1**
(43) Date of publication of application: **04.02.2015**
(21) Application number: 12873366.4
(22) Date of filing: 30.03.2012
(51) Int. Cl.: G06Q 50/02

(54) **STEP COUNT CORRECTION METHOD AND STEP COUNT CORRECTION DEVICE**

(71) Applicant: FUJITSU LIMITED, Kawasaki-shi Kanagawa 211-8588 (JP)
(72) Inventor: UCHINO, Tetsuya, Fukuoka-shi Fukuoka 814-8589 (JP); MOTOSHIMA, Toshimi, Fukuoka-shi Fukuoka 814-8589 (JP); KANAMORI, Akihito, Fukuoka-shi Fukuoka 814-8589 (JP); INENAGA, Mitsuhisa, Fukuoka-shi Fukuoka 814-8589 (JP); WATANABE, Katsuyoshi, Kawasaki-shi Kanagawa 211-8588 (JP); YAMANO, Daiji, Kawasaki-shi Kanagawa 211-8588 (JP)
(74) Representative: Cooper-Rolfe, Elizabeth Louise
(86) International application number: PCT/JP2012/058751
(87) International publication number: WO 2013/145322

(57) **Abstract**

On a farm (F), area (J1) and area (J2) are set. In area (J1), a relay device (102a) is provided and in area (J2), a relay device (102b) is provided. A step count correcting apparatus (103), through either relay device (102), receives from a communications device (101) attached to a livestock animal (A), measurement result information indicating measurement results of a step count of the livestock animal (A). The step count correcting apparatus (103), upon receiving the measurement result information, identifies from the relay device (102) that relayed the received measurement result information, the area where the livestock animal (A) is pastured. The step count correcting apparatus (103), upon identifying the area where the livestock animal (A) is pastured, uses an average correction value and/or vegetation and plant correction value specific to the identified area, to correct the measured step count of the livestock animal (A). The step count correcting apparatus (103) transmits to a client apparatus (104), information indicating the corrected step count

## Description

### TECHNICAL FIELD

The present invention relates to a step count correcting method and a step count correcting apparatus.

### BACKGROUND ART

Conventionally, a technique of attaching a pedometer to livestock such as grazing cattle and measuring the number of steps walked to detect changes in the state of the animal, such as the onset of estrus or illness is known. In this technique, the pedometer attached to the animal notifies a farm worker of measurement results at a given interval. From the measurement results notified by the pedometer, the farm worker knows that a change has occurred with an animal and considers a measure, such as mating, administering medical treatment, etc., for the change.

Patent Document 1: Japanese Laid-Open Patent Publication No. 2002-366709
Patent Document 2: Japanese Laid-Open Patent Publication No. 2003-303270

### DISCLOSURE OF INVENTION

### PROBLEM TO BE SOLVED BY THE INVENTION

Nonetheless, in a large pasture area, there are flat areas, inclined areas, and accordingly, the environment of each area included in the pasture area differs. For example, pasture areas exist of sizes that cover several kilometers on a side. In such large pasture areas, measured livestock step counts vary according to the terrain walked on in the pasture area. For example, measured livestock step counts in an inclined portion tend to increase compared to measured step counts in a flat portion. Therefore, with the conventional techniques, if livestock changes are to be detected from measured livestock step counts, variations in measurement results consequent to differences in the terrain of areas in the pasture area arise and the accuracy in detecting livestock changes may drop.

To solve the problems of the conventional techniques described above, one object of the present invention is to provide a step count correcting method and a step count correcting apparatus that can improve accuracy when detecting a specific livestock animal from a livestock step count.

### MEANS FOR SOLVING PROBLEM

To solve the above problems and achieve an object, according to one aspect of the present invention, a step count correcting method and a step count correcting apparatus that correct a step count measurement result received, via a relay device, from a step counting device attached to a livestock animal are proposed that obtain at a specific interval, the step count measurement result of the livestock animal and information of a specific relay device used to report the step count measurement result; identify a specific area correction value that corresponds to the specific relay device, by referring to the relay device and an area correction value correlated with a communications area of the relay device; correct the step count measurement result based on the specific area correction value; and output the corrected step count measurement result.

Further, to solve the above problems and achieve an object, according to one aspect of the present invention, a step count correcting method and a step count correcting apparatus that determine estrus, based on a step count measurement result from a step counting device attached to a livestock animal are proposed that obtain the step count measurement result of the livestock animal and information of a specific relay device used to report the step count measurement result; identify a specific area correction value that corresponds to the specific relay device, by referring to a correspondence relation of the relay device and an area correction value that corresponds to a communications area of the relay device; determine estrus of the livestock animal, based on the specific area correction value and the step count measurement result; and output identification information of the livestock animal determined to be in estrus.

### EFFECT OF THE INVENTION

According to one aspect of the present invention, the accuracy when detecting a specific livestock animal from the livestock step count can be improved.

### BRIEF DESCRIPTION OF DRAWINGS

FIG. 1 is a diagram depicting one example of a step count correcting method of an embodiment;
FIG. 2 is a diagram depicting example of system configuration of a step count correcting system 200;
FIG. 3 is a block diagram depicting a hardware configuration example of a communications device of the present embodiment;
FIG. 4 is a block diagram depicting a hardware configuration example of a relay device 102;
FIG. 5 is a block diagram depicting a hardware configuration example of a step count correcting apparatus 103, etc.;
FIG. 6 is a diagram depicting an example of the contents of a measurement result information table 201;
FIG. 7 is a diagram depicting an example of the contents of the relay device DB 202;
FIG. 8-1 is a diagram depicting an example of the contents of an area DB 203-1;
FIG. 8-2 is a diagram depicting an example of the contents of an adjacent area correction value DB 203-2;
FIG. 9 is a diagram depicting an example of the contents of a vegetation and plants DB 204;
FIG. 10 is a diagram depicting an example of the contents of the step count DB 205;
FIG. 11 is a block diagram depicting a functional configuration example of the step count correcting apparatus 103;
FIG. 12 is a diagram depicting one example of display by a client apparatus 104;
FIG. 13 is a flowchart depicting an example of a process performed by the communications device 101;
FIG. 14 is a flowchart (part 1) depicting an example of a process performed by the step count correcting apparatus 103; and
FIG. 15 is a flowchart (part 2) depicting the example of the process performed by the step count correcting apparatus 103.

### BEST MODE(S) FOR CARRYING OUT THE INVENTION

Embodiments of a step count correcting method and a step count correcting apparatus according to the present invention will be described in detail with reference to the accompanying drawings.

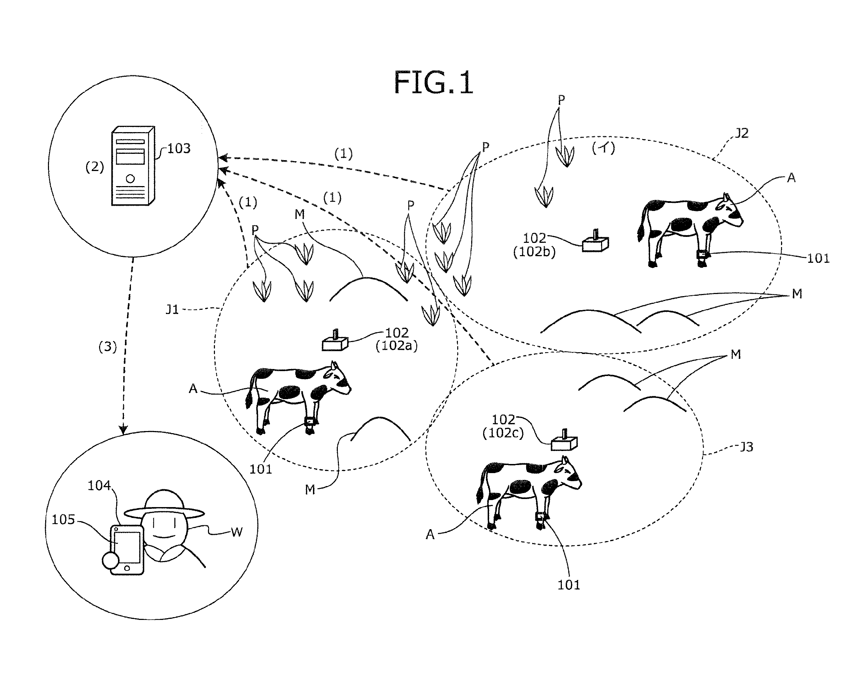

FIG. 1 is a diagram depicting one example of the step count correcting method of the present embodiment. In FIG. 1, within the premises of a farm F managed by a worker W, livestock animals A are raised. Here, the worker W is one engaged in the livestock industry. The farm F is a facility that has a pasture area for pasturing the livestock animals A. For example, as depicted in FIG. 1, the farm F has areas J1, J2, J3, which are communications areas of relay devices 102a to 102c, respectively.

The areas J1 to J3 are facilities for pasturing the livestock animals A. For example, in the area J1 and the area J2, vegetation and plants P serve as feed for the livestock animals A grow and in particular, at an adjacent point of the area J1 and the area J2, the vegetation and plants P are dense. Further, for example, although hills M are present in each of the areas J1 to J3, in particular, many hills M are present at an adjacent point of the area J2 and the area J3.

A livestock animal A is an animal that can move within the premises of the farm F, for example, within the pasture area of the farm F. For example, a livestock animal A is an animal that moves by ambulation, such as a cow, pig, horse, etc. Further, a communications device 101 is attached to each livestock animal A. Here, the communications device 101 is a portable computer that measures a step count of the livestock animal A.

For example, when the livestock animal A walks, the livestock animal A steps with the right-front leg, sending the leg out toward the ground in the direction of travel. Upon planting the right-front leg on the ground, next, the livestock animal A steps with the left-front leg, sending the leg out toward the ground in the direction of travel. Upon planting the left-front leg on the ground, again, the livestock animal A steps with the right-front leg, and repeats this series of actions. In other words, the step count of the livestock animal A can be the number of times that the livestock animal A steps with the right-front leg or the left-front leg, sending the leg out toward the ground in the direction of travel.

For example, when the state of the livestock animal A becomes an abnormal state, which differs from a normal state, consequent to a state change of the livestock animal A, such as the onset of estrus or illness, giving birth, the appearance of a natural predator, etc., the step count per unit time of the livestock animal A increases or decreases compared to that for the normal state. Here, estrus is an excited state accompanying reproductive activity of the livestock animal A. Illness is a state in which the physical or emotional state of the livestock animal A is poor or unfavorable.

When an abnormal state of the livestock animal A arises, the step count measured from the livestock animal A changes. The worker W can know that a change has occurred with the livestock animal A from the step count measurement result of the livestock animal A.

The communications device 101 is capable of communicating with multiple relay devices 102 installed within the premises of the farm F. Here, a relay device 102 is a computer that is capable of communicating with the communications device 101 and a step count correcting apparatus 103 described hereinafter. Each of the relay devices 102 is installed at a different position in the farm F. The communications device 101, via a communicable relay device 102, transmits to the step count correcting apparatus 103, measurement result information that indicates measurement results.

For example, when the communications device 101 is located in the area J1, the communications device 101 can communicate with a relay device 102a indicated as an example in FIG. 1 by reference numeral 102a. In other words, when a livestock animal A is pastured in the area J1, the communications device 101 attached to the livestock animal A can communicate with the relay device 102a. Further, when the communications device 101 is located in the area J2, the communications device 101 can communicate with a relay device 102b indicated as an example in FIG. 1 by reference numeral 102b. In other words, when livestock animal A is pastured in the area J2, the communications device 101 attached to the livestock animal A can communicate with the relay device 102b.

The step count correcting apparatus 103 is a computer connected to the relay devices 102, and via the relay devices 102, is able to receive measurement result information 110 transmitted by the communications device 101. Further, for example, the step count correcting apparatus 103 is communicably connected to a client apparatus 104, via a given line.

The client apparatus 104 is a portable computer having a display 105 and capable of communicating with the step count correcting apparatus 103. The client apparatus 104, upon receiving information from the step count correcting apparatus 103, displays images based on the received information, on the display 105.

Here, as a technique of finding an abnormal state of a pastured livestock animal A, for example, an example will be given describing a technique of finding an estrus state of a female cow. When in estrus, the behavior of a female cow becomes active. If the worker W is in the vicinity of the female cow, the worker W visually confirms the state of the female cow and thereby, can know whether the female cow is in estrus. However, since the worker W has other work such as cleaning the cattle shed and the pasture area is large, the worker W cannot always check the state of the female cow.

If a female cow enters estrus when the worker W is not around, the worker W may not realize that the female cow has entered estrus, losing an opportunity to mate the female cow having a possibility of becoming impregnated. The loss of an opportunity to mate a female cow having a possibility of becoming impregnated is a financial loss for the worker W and/or the manager of the farm F.

When pasturing female cows, the worker W may also pasture stud bulls for natural mating to occur. However, natural mating by a stud bull is not desirable from the viewpoint of current livestock management ideals of producing better offspring. Furthermore, since bulls are aggressive, only an experienced worker W can handle the bulls and thus, is not an effective technique.

Further, a technique is present that uses the characteristic that when a female cow is in estrus, the behavior of the female cow becomes more active and naturally, the step count increases. The technique involves attaching a pedometer to female cows and periodically notifying the worker W of the step counts of the female cows. Nonetheless, the step count of a female cow increases and decreases consequent to other factors in addition to an abnormal state such as estrus.

In addition to an abnormal state, the step count of a female cow may be affected by other factors such as the terrain of the pasture area and the vegetation and plants growing in the pasture area. Since the pasture area is assumed to require a large area, the pasture area is created by clearing mountains and forests. Therefore, differences in the terrain of each area within the pasture area may occur such as an area covered by flat terrain being large and a sloped area being wide.

For example, when a cow is walking on an inclined surface, the feet of the cow slip and therefore, the distance traveled by one step decreases. Further, when a cow is walking downhill, the cow gains momentum from the effects of gravity. As a result, the step count of a cow measured when the cow is pastured in a pasture area having a wide area covered by sloped terrain tends to increase more than the step count measured when the cow is pastured in a pasture area having a wide area covered by flat terrain.

Further, although vegetation and plants may be present in the pasture area, the vegetation and plants cover a myriad of varieties. The vegetation and plants, such as legumes, panicium grasses, etc., growing in each of the areas, like the terrain, also differ. When a cow is walking in an area having vegetation, the vegetation becomes caught on the feet of the cow, making it difficult for the cow to walk. The size of leaves and stems on the ground varies according to the type of plant and the larger the plants are, the more ambulation of the cow is impeded. As a result, the larger the vegetation and plants are in an area, the more the measured step count of the cow when pastured tends to decrease.

Therefore, since the measured step count of a female cow varies not only consequent to estrus, but also consequent to the terrain, vegetation and plants in the area, the accuracy of detection drops in a case where estrus is to be detected from the step count of a female cow. As a result, depending on the area in which the female cow walked, the worker may not detect estrus of the female cow or may detect the female cow to be in estrus although the female is actually not in estrus.

Cases where estrus of the female cow fails to be detected become financial losses for the worker W and/or the manager of the farm F. In cases where the female cow is detected to be in estrus although the female cow is actually not in estrus, the worker W goes to a female cow that does not require attending to, increasing the work load. Further, to make the terrain, vegetation and plants uniform in each area to prevent, among the areas, variations in the step counts of the cows, requires a huge cost and is not desirable from the perspective of livestock farming.

Thus, the step count correcting method of the present embodiment identifies the terrain of the area in the pasture area, from the relay device 102 that relayed the communication from the communications device 101 attached to a livestock animal A. By converting the step count of the livestock animal A subject to variation consequent to the terrain of the area in the pasture area in which the livestock animal A is pastured, the step count correcting method of the present embodiment increases the accuracy of detection when a livestock animal A in estrus is detected from the step count of the livestock animal A.

Hereinafter, one example of the step count correcting method of the embodiment will be described. In the present embodiment, estrus will be taken as one example of an abnormal state of the livestock animal A. Further, in the present embodiment, to make the description of the invention clear, the livestock animal A will be described as a female cow. Female cows have a characteristic in that when in estrus, the step count per unit time increases compared to periods when not in estrus and this characteristic of female cows is used.

(1) The communications device 101 measures the step count of the livestock animal A and at a given transmission interval, transmits to the step count correcting apparatus 103, via a relay device 102 installed in the pasture area, measurement result information indicating measurement results of the step count for the livestock animal A. Here, as one example, the communications device 101 transmits the measurement result information 110 at a one-hour transmission interval.
(2) The step count correcting apparatus 103, via the relay device 102, receives the measurement result information transmitted by the communications device 101, and obtains the step count of the livestock animal A measured by the communications device 101. Further, the step count correcting apparatus 103 identifies from the relay device 102 that relayed the received measurement result information, the area in which the livestock animal A was pastured in the pasture area when the step count was measured. For example, here, as depicted by ( 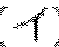 ) in FIG. 1, the area J2 in the pasture area is assumed to be identified as the area where the livestock animal A was when the step count was measured. As described above, here, the area J2 is an area within the pasture area and has vegetation and plants P, and hills M, etc.
   If the livestock animal A remains moving about in the area J2 when the step is measured, the step count correcting apparatus 103 uses an average correction value preliminarily prepared for the area J2 and corrects the step count measured by the communications device 101. A concrete correction example for correction by terrain will be described hereinafter.
(3) The step count correcting apparatus 103 transmits the corrected step count of the livestock animal A to the client apparatus 104. The client apparatus 104 displays on the display 105, an image based on the corrected step count received from the step count correcting apparatus 103. From the display contents of the display 105, the worker W can know the corrected step count of the livestock animal A. Therefore, the worker W can know that the variation consequent to terrain is a reduced step count of the livestock animal A, whereby the accuracy when detecting estrus from the step count of the livestock animal A can be increased.

As described above, the step count correcting apparatus 103 identifies from the relay device 102 that relayed the communication from the communications device 101 attached to the livestock animal A, the terrain of the area in which the livestock animal A is pastured in the pasture area, and converts into a step count for standard terrain, the livestock animal A step count subject to variation consequent to the terrain in the area in which the livestock animal A is pastured in the pasture area. Therefore, the step count correcting apparatus 103 can suppress variations in the step count of the livestock animal A consequent to the terrain and increase the accuracy when a livestock animal A in estrus is detected from the step count of the livestock animal A.

### (Example of system configuration of step count correcting system)

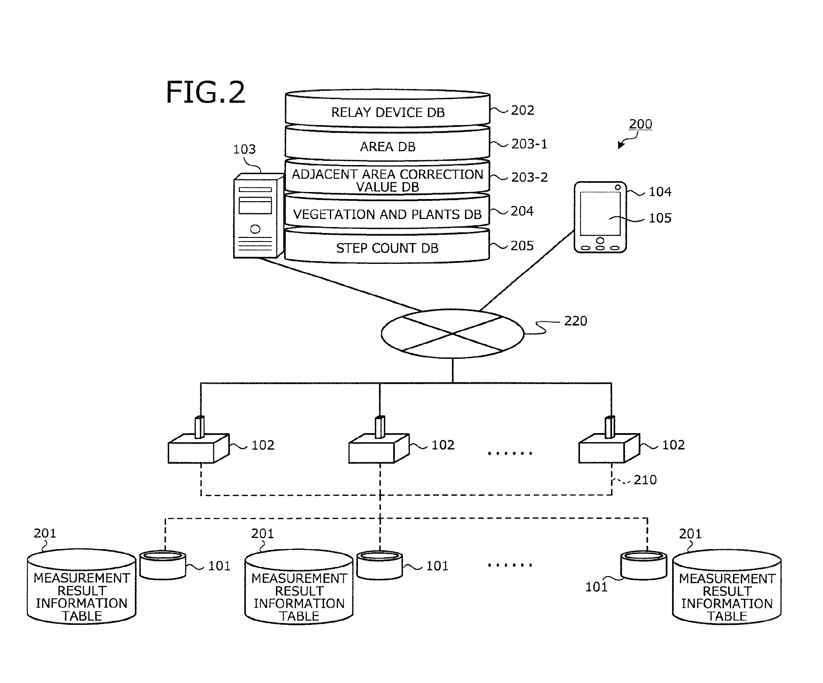

An example of system configuration of the step count correcting system of the present embodiment will be described. FIG. 2 is a diagram depicting example of system configuration of a step count correcting system 200. In FIG. 2, the step count correcting system 200 includes one or more of the communications devices 101, the relay device 102 in plural, and the step count correcting apparatus 103. Although in FIG. 2, a case in which the communications device 101 is provided in plural is depicted, a singular communications device 101 may be provided.

As depicted in FIG. 2, when the step count correcting system 200 has the communications device 101 in plural, each of the communications devices 101 transmits communications device identification information thereof when transmitting measurement result information. Here, the communications device identification information is information that can identify one communications device 101 from among plural communications devices 101. For example, the communications device identification information is information representing a communications device ID specific to each of the communications devices 101. Hereinafter, in the present embodiment, the communications device identification information will be simply referred to as "communications device ID".

In the step count correcting system 200, the communications device 101 and the relay device 102 are connected through a wireless communications network 210. The communications device 101 and the relay device 102 each have a given range around the device 101, 102 (e.g., a range of a 150-meter radius around the device 101, 102), as a communications area in which communication through the wireless communications network 210 is possible. When having a positional relation enabling communication, the communications device 101 and the relay device 102 are connected by the wireless communications network 210. For example, existing wireless communication enabling a communication distance of 50 meters to 200 meters can be applied as the wireless communications network 21.

Further, the relay device 102, the step count correcting apparatus 103, and the client apparatus 104 are connected by a network 220. For example, the network 220 is the Internet, a local area network (LAN), a wide area network (WAN), etc.

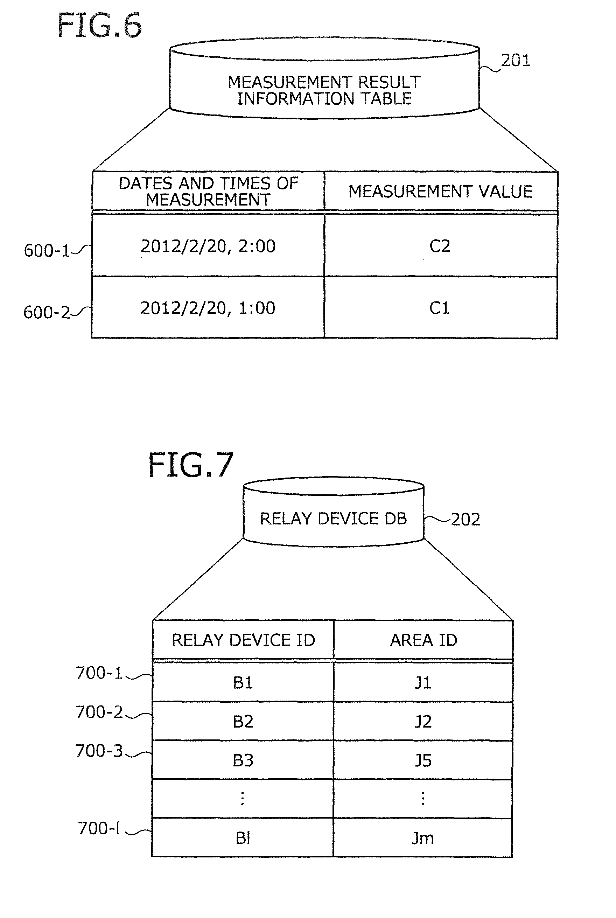

The communications device 101 has a measurement result information table 201 and is a portable computer attached to livestock animals A raised on the farm F. The communications device 101 has a function of measuring the step count of the livestock animal A to which the communications device 101 is attached and a communications function through the wireless communications network 210. For example, a pedometer with an additional function of communication via the wireless communications network 210 is applicable as the communications device 101. Contents of the measurement result information table 201 will be described with reference to FIG. 6.

The relay device 102 is installed in each area of the pasture area of the farm F and is a computer having a communications function via the wireless communications network 210 and a communications function via the network 220. The relay devices 102 are respectively installed at a different installation position.

The step count correcting apparatus 103 has a relay device DB 202, an area DB 203-1, an adjacent area correction value DB 203-2, a vegetation and plants DB 204, and a step count DB 205, and is a computer having a communications function via the network 220. For example, For example, a server included in a cloud computing system, a personal computer (PC), a note PC, etc. used by a manager of the farm F or the worker W are applicable as the step count correcting apparatus 103. The contents of the relay device DB 202, the area DB 203-1, the adjacent area correction value DB 203-2, the vegetation and plants DB 204, and the step count DB 205 will be described hereinafter with reference to FIGs. 7 to 10.

The client apparatus 104 is a computer having the display 105 that displays images based on various types of information, and a communications function via the network 220. For example, a PC or note PC, a mobile telephone, a smartphone, and the like used by the worker W of the farm F are applicable as the client apparatus 105.

### (Hardware configuration example of communications device)

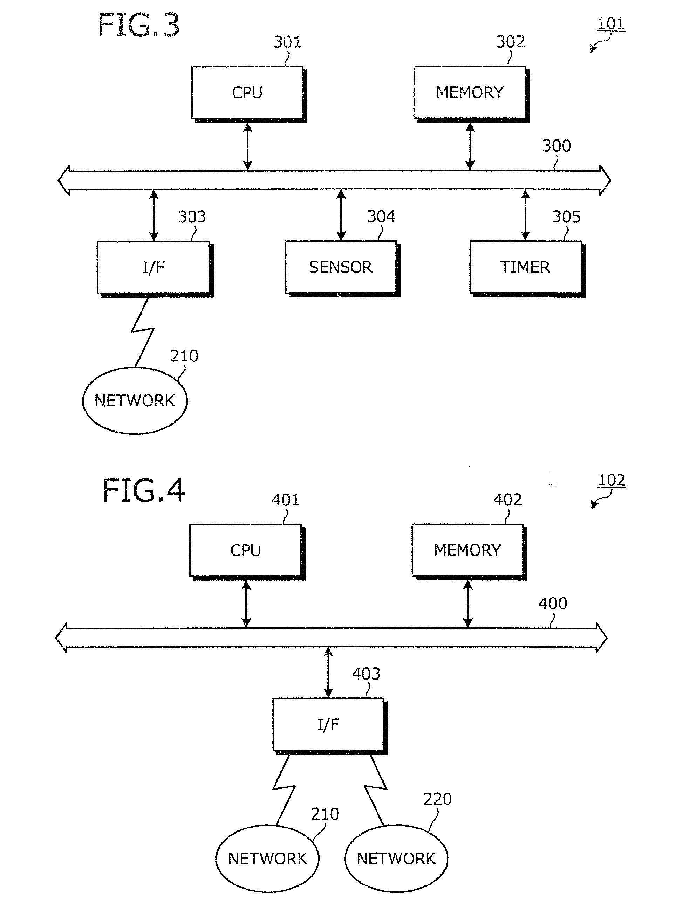

A hardware configuration example of the communications device 101 will be described. FIG. 3 is a block diagram depicting a hardware configuration example of the communications device of the present embodiment. In FIG. 3, the communications device 101 includes a central processing unit (CPU) 301, memory 302, an interface (I/F) 303, a sensor 304, and a timer 305, respectively connected by a bus 300.

Here, the CPU 301 governs overall control of the communications device 101. The memory 302 includes read-only memory (ROM), random access memory (RAM), and flash ROM. The ROM and the flash ROM, for example, store various types of programs such as a boot program. The RAM is used as a work area of the CPU 301.

The I/F 303 is connected to the wireless communications network 210 through a communications line and is connected to other apparatuses such as the relay devices 102, via the wireless communications network 210. The I/F 303 administers an internal interface with the wireless communications network 210 and controls the input and output of data from external apparatuses.

The sensor 304 outputs information for detecting behavior of the communications device 101. For example, when the sensor 304 is implemented by a gyroscope or a triaxial accelerometer and the communications device 101 accelerates, the sensor 304 outputs information according to the acceleration. The timer 305 has a function of measuring time. For example, the timer 305 is implemented by a real time clock (RTC) and measures the actual time. Further, the timer 305 may measure the time that elapses from a given time point. Configuration may be such that the timer 305 is disposed external to the communications device 101 and the communications device 101 obtains the measurement results of the timer 305 through the wireless communications network 210.

### (Hardware configuration example of relay device)

A hardware configuration example of the relay device 102 will be described. FIG. 4 is a block diagram depicting a hardware configuration example of the relay device 102. In FIG. 4, the relay device 102 includes a CPU 401, memory 402, and an I/F 403, respectively connected by a bus 400.

Here, the CPU 401 governs overall control of the relay device 102. The memory 402 includes ROM, RAM, and flash ROM. The ROM and flash ROM, for example, store various types of programs such as a boot program. The RAM is used as a work area of the CPU 401.

The I/F 403 is connected to the wireless communications network 210 through a communications line and is connected to other apparatuses such as the communications device 101, via the wireless communications network 210. The I/F 403 is further connected to the network 220 through a communications line and through the network 220, is connected to other apparatuses such as the step count correcting apparatus 103. The I/F 403 administers an internal interface with the wireless communications network 210 and the network 220; and controls the input and output of data from external apparatuses.

### (Hardware configuration example of step count correcting apparatus and client apparatus)

A hardware configuration example of the step count correcting apparatus 103 and the client apparatus 104 will be described. Here, the step count correcting apparatus 103 and the client apparatus 104 will be indicated as simply "the step count correcting apparatus 103, etc.".

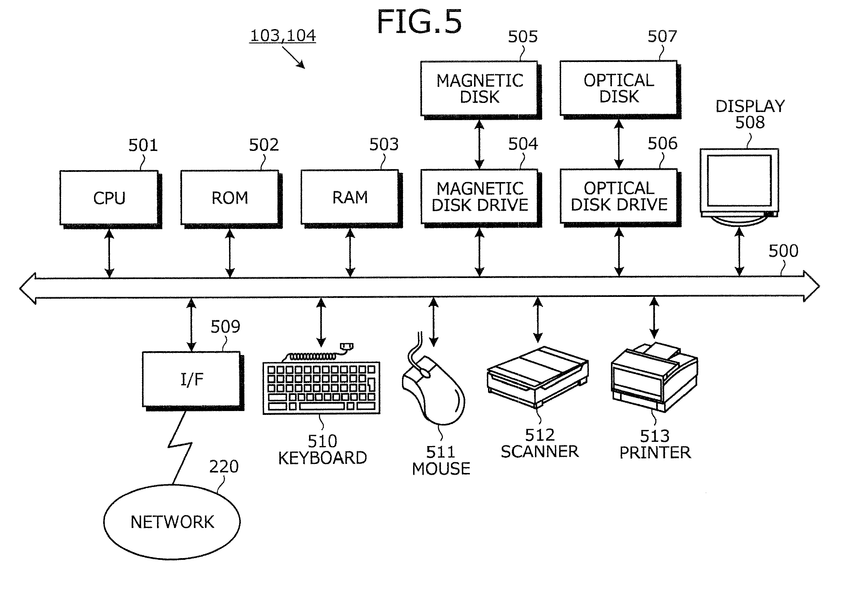

FIG. 5 is a block diagram depicting a hardware configuration example of the step count correcting apparatus 103, etc. In FIG. 4, the step count correcting apparatus 103, etc. include a CPU 501, ROM 502, RAM 503, a magnetic disk drive 504, a magnetic disk 505, an optical disk drive 506, an optical disk 507, a display 508, an I/F 509, a keyboard 510, a mouse 511, a scanner 512, and a printer 513, respectively connected by a bus 500.

Here, the CPU 501 governs overall control of the step count correcting apparatus 103, etc. The ROM 502 stores programs such as a boot program. The RAM 503 is used as a work area of the CPU 501. The magnetic disk drive 504, under the control of the CPU 501, controls the reading and writing of data with respect to the magnetic disk 505. The magnetic disk 505 stores data written thereto under the control of the magnetic disk drive 504.

The optical disk drive 506, under the control of the CPU 501, controls the reading and writing of data with respect to the optical disk 507. The optical disk 507 stores data written thereto under the control of the optical disk drive 506, the data being read out by a computer.

The display 508 displays documents, images, and functional information in addition to a cursor, icons, and toolboxes. A CRT, TFT liquid crystal display, plasma display, and the like may be employed as the display 508.

The I/F 509 is connected to the network 220 through a communications line, and is connected to other apparatuses such as the relay device 102 and the client apparatus 104, via the network 220. The I/F 509 administers an internal interface with the network 220, and controls the input and output of data from external apparatuses. A modem, LAN adapter, etc. may be employed as the I/F 509.

### The keyboard 510 has keys for inputting text, numerals, various instructions, etc. and inputs data. The keyboard 510 may be a touch panel input pad, a numeric pad, etc. The mouse 511 is used to move the cursor, select a range, move or change the size of a window, etc. As long as functions identical to a pointing device are provided, a trackball, a joy stick, and the like may be employed.

The scanner 512 optically reads images and takes in image data into the step count correcting apparatus 103. The scanner 512 may have an optical character reader (OCR) function. The printer 513 prints image data and document data. A laser printer or ink jet printer may be employed as the printer 513.

Further, for example, among the components described above, the step count correcting apparatus 103 may be configured to omit the optical disk drive 506, the optical disk 507, the display 508, the mouse 511, the scanner 512, and the printer 513. The client apparatus 104 may be configured to omit the optical disk drive 506, the optical disk 507, the mouse 511, the scanner 512, and the printer 513.

### (Example of information stored by communications device)

An example of the information stored by the communications device 101 will be described. As described above, the communications device 101 stores the measurement result information table 201. For example, the measurement result information table 201 is implemented by the memory 302 of the communications device 101.

### <Example of contents of measurement result information table>

FIG. 6 is a diagram depicting an example of the contents of the measurement result information table 201. In FIG. 6, the measurement result information table 201 has fields for dates and times of measurement, and measurement values. By setting information into these fields, measurement result information for each combination of a measurement value and the date and time of the measurement is stored as a record in the measurement result information table 201. In the example depicted in FIG. 6, records of measurement result information 600-1 to 600-6 are stored in the measurement result information table 201.

Here, the date and time of measurement represents the date and time of past transmissions of measurement result information. In the case of the present embodiment, as one example, the date and time of measurement indicates the date and time of the two most recent transmissions of the measurement result information. The measurement value represents a past measurement value of the step count of the livestock animal A at the time of transmission of the measurement result information. In the case of the present embodiment, as one example, the measurement values of the step counts of the livestock animal A at the time of the two most recent transmissions of the measurement result information are indicated.

For example, the communications device 101 cumulates, as the current measurement value, the step count of the livestock animal A from the time when the measurement value is set to "0" until the current time. With each step that the livestock animal A takes, the communications device 101 instantaneously accelerates. Upon detecting this acceleration via the sensor 304, the communications device 101 increments the current measurement value by "+1".

When the transmission time for the measurement result information arrives according to the measurement result of the timer 305, the communications device 101 stores measurement result information in which the current measurement value is correlated with the date and time of measurement for this transmission. If the transmission time for the measurement result information is a one-hour interval, for example, transmission occurs every hour, on the hour.

For example, in FIG. 6, the measurement result information 600-1 indicates that the measurement value at 2:00 on 2012/02/20 is "C2". Here, "C2" is a positive integer. Upon storing the measurement result information to the measurement result information table 201, the communications device 101 transmits to the step count correcting apparatus 103, via the relay device 102, each record of measurement result information stored in the measurement result information table 201. The relay device 102 appends the relay device ID thereof to the measurement result information and transmits the measurement result information to the step count correcting apparatus 103. In the example depicted in FIG. 6, the communications device 101 transmits the measurement result information 600-1, 600-2 to the relay device 102.

Each time the transmission time for the measurement result information arrives, the communications device 101 transmits the measurement value at the previous transmission and the measurement value at the current transmission, thereby enabling the step count correcting apparatus 103 to stably obtain measurement results for the communications device 101. For example, even if the step count correcting apparatus 103 cannot receive the measurement result information previously transmitted from the communications device 101, the step count correcting apparatus 103 can obtain the measurement value for the previous transmission at the current transmission time.

Although an example has been described where the communications device 101 stores measurement result information in which the two most recent transmission times and measurement value are correlated, configuration is not limited hereto. The communications device 101 may store three records of the measurement result information in which the three most recent transmission times and corresponding measurement values are correlated. For instance, in this case, in the example depicted in FIG. 6, a measurement value at "2012/2/20, 0:00" is stored. The communications device 101 may store three or more records of the measurement result information. The more measurement result information transmitted by the communications device 101 at one transmission, the more assuredly the step count correcting apparatus 103 can receive the measurement value for each transmission time.

### (Example of information stored by step count correcting apparatus)

Examples of the contents of the various DBs 202, 203, 204, and 205 stored by the step count correcting apparatus 103 will be described. For example, in the description hereinafter, the various DBs 202, 203, 204, and 205 are implemented by a memory apparatus such as the ROM 502, the RAM 503, the magnetic disk 505, and the optical disk 507 of the step count correcting apparatus 103.

### <Example of contents of relay device DB 202>

FIG. 7 is a diagram depicting an example of the contents of the relay device DB 202. As depicted in FIG. 7, the relay device DB 202 has fields for relay device IDs and area IDs. By setting information into these fields, installation area information for each combination of a relay device ID and an area ID is stored as a record in the relay device DB 202. In the example depicted in FIG. 7, records of the installation area information 700-1 to 700-1 are stored in the relay device DB 202.

Here, a relay device ID is information representing an identifier specific to each of the relay devices 102. An area ID is information representing an identifier specific to each of the areas in which the relay devices 102 are installed. For example, in FIG. 7, installation area information 700-1 indicates that the relay device 102 of the relay device ID "B1" is installed in the area J1.

### <Example of contents of area DB 203-1>

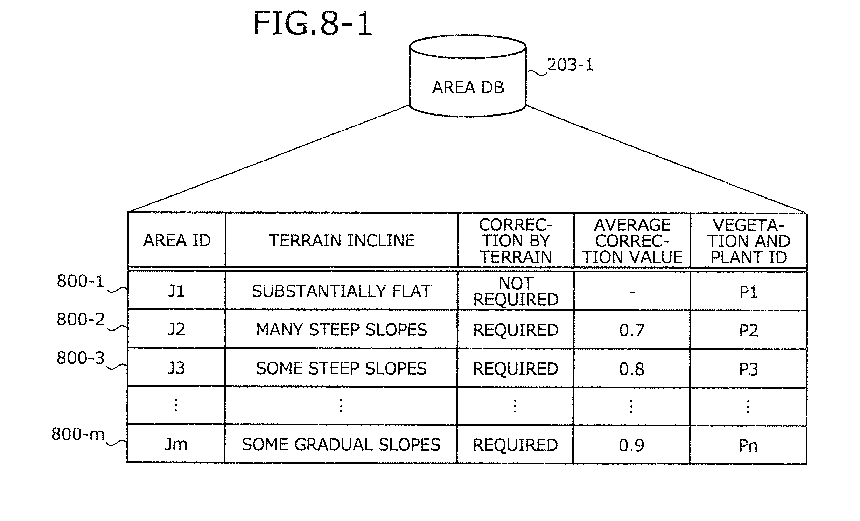

FIG. 8-1 is a diagram depicting an example of the contents of the area DB 203-1. As depicted in FIG. 8-1, the area DB 203-1 has fields for area IDs, specifying whether correction by terrain is required, average correction values, and vegetation and plant IDs. By setting information into these fields, area information for each combination of an area ID, specification of whether correction by terrain is required for the area, an average correction value, and a vegetation and plant ID as a record in the area DB 203-1. In the example depicted in FIG. 8-1, records of area information 800-1 to 800-m are stored in the area DB 203-1. Further, in FIG. 8-1, for the purposes of description, the terrain of each area is indicated to have slopes.

Here, an area ID represents an identifier specific to each of the areas J1 to Jm. Specification of whether correction by the terrain is required is information indicating whether the step count measured in the area J1 to Jm is to be corrected by the terrain. Specification of whether correction by the terrain is required is preliminarily determined by the worker W, based on the incline of the terrain in the areas J1 to Jm. This correction is used when a target livestock animal A remains within one area for a given period and does not move to another area.

For example, as depicted in FIG. 8-1, the incline of the terrain of the area J1 is "substantially flat", indicating that the terrain in the area J1 is mainly terrain that is substantially flat. Therefore, the step count of the livestock animal A is not likely to increase or decrease consequent to the effect of the terrain in the area J1. As a result, for the area J1, specification of whether correction by the terrain is required is set to "not required".

On the other hand, as depicted in FIG. 8-1, the incline of the terrain in the area J2 has "many steep slopes", indicating that terrain having steep slopes is prevalent in the area J2. Therefore, the step count of the livestock animal A is likely to increase consequent to the effect of the terrain in the area J2. More specifically, when the livestock animal A walks uphill, the legs of the livestock animal A slip, decreasing the distance traveled by one step of the livestock animal A. Further, when the livestock animal A walks downhill, the livestock animal A gains momentum from the effects of gravity. Therefore, the step count of the livestock animal A is likely to increase consequent to the effect of the terrain in the area J2.

Thus, for the area J2, specification of whether correction by the terrain is required is set to "required". Further, similar to the area J2, for the areas J3 and Jm having slopes, the step count of the livestock animal A is likely to increase consequent to the effects of the terrain in the respective areas. Therefore, specification of whether correction by the terrain is required for the areas J3 and Jm is set to "required".

The average correction value is information specifying a correction value t be used when a step count is corrected by the terrain, for an area that is in the pasture area and for which correction by the terrain is "required". The average correction value is preliminarily determined by the worker W, according to the incline of the terrain in the respective areas J1 to Jm. For example, the step count correcting apparatus 103 multiplies the step count of the livestock animal A obtained from measurement results of the communications device 101 before correction, by the average correction value to calculate the corrected step count that has been corrected for the terrain on which the livestock animal A moves in the area.

Thus, when the corrected step count is less than the step count before correction by the correction for the terrain, the average correction value is a value less than "1.0". On the contrary, when the corrected step count is greater than the step count before correction, the average correction value is a value greater than "1.0". Hereinafter, the step count before correction will be indicated as "pre-correction step count" and the corrected step count will be indicated as "post-correction step count".

For example, as described above, the step count of the livestock animal A tends to increase more on terrain having slopes than on flat terrain. Therefore, for an area having slopes, an average correction value of a value less than "1.0" is set. For an area having many slopes, an average correction value of a small value is set. Further, the steeper a slope is, the greater the effect of the terrain is. More specifically, when a slope is steep, the feet of the livestock animal A significantly slip and the livestock animal A further gains momentum. Therefore, for areas with steep slopes, an average correction value of a small value is set.

In the example depicted in FIG. 7, the average correction value for the area J2, whose terrain incline has "many steep slopes", is the smallest value of "0.7". Further, the average correction value for the area J3, whose terrain incline has "some steep slopes", is the next smallest value after the average correction value for the area J2, "0.8". The average correction value for the area Jm, whose terrain incline has "some gradual slopes", is "0.9".

A vegetation and plant ID is an identifier of the vegetation and plants P1 to Pn, representing the vegetation and plants growing in the areas J1 to Jm, respectively. In the example depicted in FIG. 8-1, the vegetation and plants P1 are growing in the area J1 and the vegetation and plants P2 are growing in the area J2.

### <Example of contents of adjacent area correction value DB 203-2>

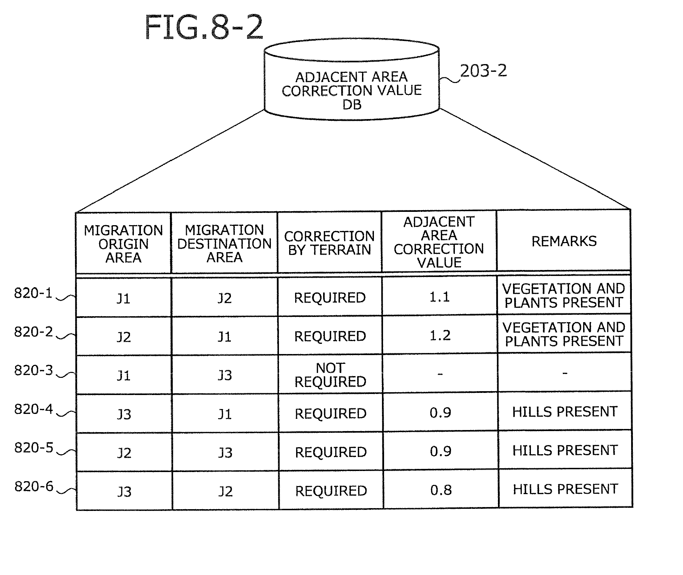

FIG. 8-2 is a diagram depicting an example of the contents of the adjacent area correction value DB 203-2. As depicted in FIG. 8-2, the adjacent area correction value DB 203-2 has fields for migration origin area IDs, migration destination area IDs, specifying whether correction by terrain accompanying area change is required, and adjacent area correction values. Information is stored into these fields. In the example depicted in FIG. 8-2, migration area information 820-1 to 820-m is stored in the adjacent area correction value DB 203-2. Further, in FIG. 8-2, for the purpose of description, trends of the terrain, and vegetation and plants in the adjacent portions of each of the areas are indicated as a remark.

Here, a migration origin area ID represents an identifier specific to each of the areas J1 to Jm and, for example, indicates the area in which the livestock animal A is located at the time of the previous transmission of the measurement result information. A migration destination area ID represents an identifier specific to each of the areas J1 to Jm and, for example, indicates the area in which the livestock animal A is located at the time of the current transmission of the measurement result information.

Specification of whether correction by the terrain is required indicates whether the terrain of an adjacent portion of the area specified by the migration destination area ID and the area specified by the migration destination area ID when the livestock animal A moves from the area specified by the migration origin area ID to the area specified by the migration destination area ID, requires correction by the terrain. Further, an adjacent area correction value is information indicating whether correction by the terrain is to be performed for the adjacent terrain. Specification of whether correction by the terrain of the adjacent portion is required is preliminarily determined by the worker W, based on the incline of the terrain of the adjacent portions between the areas J1 to Jm. This correction is used when the livestock animal A has moved to be between areas.

For example, as depicted in FIG. 8-2, the migration area information 820-1 indicates that the migration origin area is J1, the migration destination area is J2, correction for the terrain of the adjacent portion that is in the migration destination area J2 and adjacent to the migration origin area J1 is "required", and the adjacent area correction value is "1.1". Further, as indicated by "vegetation and plants present" indicated as a remark, in the migration destination area J2, in the adjacent portion adjacent to the migration origin area J1, the step count of the livestock animal A has a possibility of being reduced consequent to the vegetation and plants and therefore, here correction to increase the step count is to be performed.

### <Example of contents of vegetation and plants DB 204>

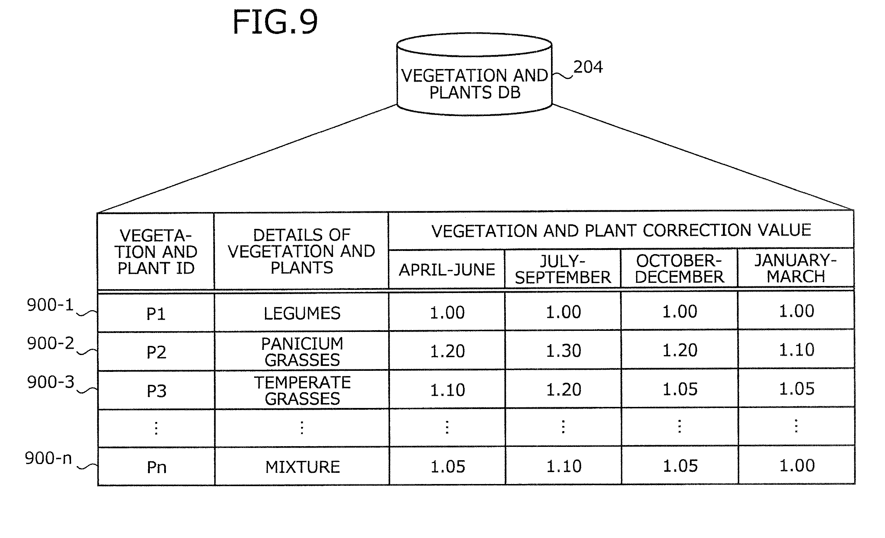

FIG. 9 is a diagram depicting an example of the contents of the vegetation and plants DB 204. As depicted in FIG. 9, the vegetation and plants DB 204 has fields for vegetation and plant IDs, and vegetation and plant correction values. By setting information into these fields, vegetation and plant information for each combination of a vegetation and plant ID and a vegetation and plant correction value is stored as a record in the vegetation and plants DB 204. In the example depicted in FIG. 9, records of the vegetation and plant information 900-1 to 900-n are stored in the vegetation and plants DB 204. Further, in FIG. 9, for the purpose of description, details of the respective vegetation and plants are indicated.

Here, a vegetation and plant ID represents an identifier specific to each of the vegetation and plants P1 to Pn. A vegetation and plant correction value represents a correction value used to correct for the vegetation and plants. The vegetation and plant correction value is preliminarily determined by the worker W, based on vegetation and plant details of the vegetation and plants P1 to Pn. For example, the step count correcting apparatus 103 multiplies the pre-correction step count by the vegetation and plant correction value to calculate the post-correction step count.

Thus, when the post-correction step count is corrected by the vegetation and plants to be less than the pre-correction step count, the vegetation and plant correction value is a value less than "1.00". On the contrary, when the post-correction step count is corrected to be greater than the pre-correction step count, the vegetation and plant correction value is a value greater than "1.00".

The step count of the livestock animal A tends to decrease more on terrain with vegetation than on terrain without vegetation. More specifically, when the livestock animal A walks on terrain with vegetation, the vegetation becomes caught on the feet of the livestock animal A, making it difficult for the livestock animal A to walk. As a result, the step count of the livestock animal A is likely to decrease. The size of leaves and stems on the ground varies according to the type of plant and the larger the plants are, the more ambulation of the livestock animal A is impeded, significantly affecting ambulation of the livestock animal A. Therefore, the larger the plants are, the larger the value of vegetation and plant correction value is set.

In the example depicted in FIG. 9, the vegetation and plant correction value for the vegetation and plants P2, whose vegetation and plant details indicate "panicium grasses", is greater than the vegetation and plant correction value for the vegetation and plants P1, whose vegetation and plant details indicate "legumes", indicating that "panicium grasses" grow to be larger than "legumes" and therefore, greatly affect ambulation of the livestock animal A, causing the step count of the livestock animal A to decrease.

Further, the vegetation and plant correction values for the vegetation and plants P1 to Pn are set to be different values according to period. For example, plants germinate from seeds and after maturing, flower and bear fruit to leave behind seeds, and die off. The germination period, growth period, dying off period differ according to the plant type. During the growth period, plants appear on the ground and grow. Therefore, during the growth period, plants have a large impact on the ambulation of the livestock animal A compared to other periods.

Thus, during the growth period of the vegetation and plants P1 to Pn, the vegetation and plant correction value is set to be greater compared to other periods. For example, the vegetation and plants P2 "panicium grasses" grows from July to September. Therefore, the vegetation and plant correction value for the vegetation and plants P2 is set to be greatest "1.30" for July to September.

On the other hand, during the period when a plant dies off, the plant, for the most part, does not remain on the ground surface and even if the plant does, only a very small portion remains. Therefore, during the season when a plant dies off, the plant has a small impact on the ambulation of the livestock animal A compared to other periods. Thus, the vegetation and plant correction value for the periods when the vegetation and plants P1 to Pn die off is set to be small compared to other periods. For example, the vegetation and plants P2 "panicium grasses" die off from January to March. Therefore, the vegetation and plant correction value for the vegetation and plants P2 is set to be smallest "1.10" from January to March. The vegetation and plants P1 "legumes" have a small impact on the ambulation of the livestock animal A throughout the entire year and therefore, the vegetation and plant correction value is set to be "1.00" throughout the year.

### <Example of contents of step count DB 205>

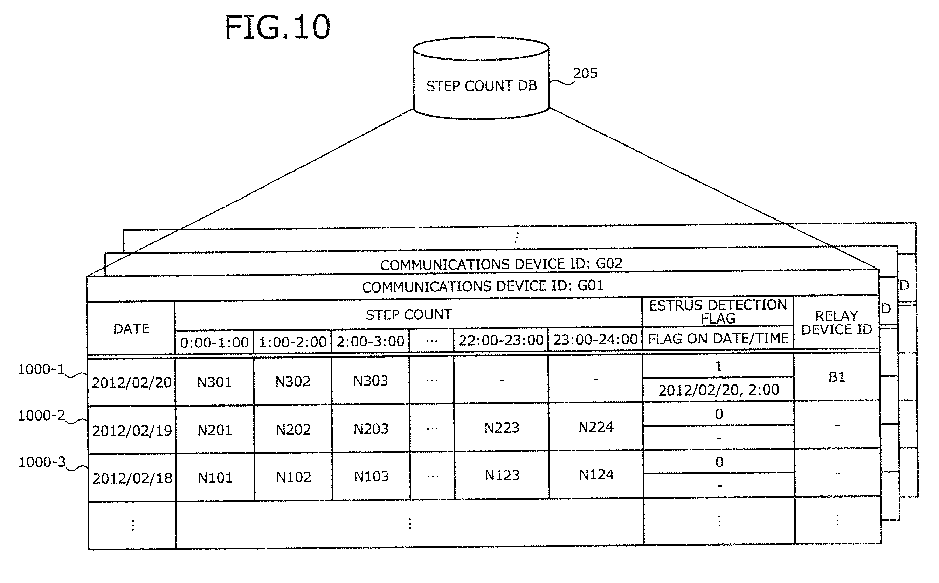

FIG. 10 is a diagram depicting an example of the contents of the step count DB 205. In FIG. 10, the step count DB 205 has fields for dates, step count, and estrus detection flags. By setting information into these fields, step count information for each combination of a date, a step count, and an estrus detection flag step is stored as a record in the count DB 205. In the example depicted in FIG. 10, records of the step count information 1000-1 to 1000-3 are stored in the step count DB 205.

Here, a date represents the date when the step count was measured and, for example, is indicated as year/month/day. The step count represents the step count of the livestock animal A. Here, in the step count field, time slot fields are prepared and for each time slot, the step count DB 205 stores a step count of the livestock animal A. In the example depicted in FIG. 10, time slot fields of one-hour intervals, e.g., "0:00 to 1:00", "1:00 to 2:00", etc. are prepared.

The values set in the time slot fields as the step counts of the livestock animal A are values obtained by correcting the measurement results measured for each time slot by the communications device 101, by the average correction values for the areas J1 to Jm and the vegetation and plant correction values for the vegetation and plants P1 to Pn. In other words, in the time slot fields, the post-correction step count described above is stored. In the example depicted in FIG. 10, in the time slot field "1:00 to 2:00" for "2012/02/20", N302 is stored and is the post-correction step count obtained by correcting the pre-correction step count of the livestock animal A from "1:00 to 2:00" on "2012/02/20" by the terrain and/or the vegetation and plants.

### (Example of information stored to time slot fields by step count correcting apparatus 103)

Here, an example of the information stored to the time slot fields by the step count correcting apparatus 103 will be described. In the description hereinafter, the pre-correction step count calculated from the measurement value of the measurement result information received by the step count correcting apparatus 103 is assume to be "X", the average correction value is assumed to be "t", and the vegetation and plant correction value is assumed to be "s". Further, the post-correction step count calculated by correcting for the terrain is assumed to be "Y", and the post-correction step count calculated by correcting for the vegetation and plants is assumed to be "Z".

The step count correcting apparatus 103 calculates based on the received measurement result information, a pre-correction step count of the livestock animal A, for each time slot. For example, the received measurement result information includes information indicating that the measurement value at the time point 2012/02/20, 2:00 is "C2", and the measurement value at the time point 2012/02/20, 1:00 is "C1". C2 and C1 are respectively positive integers. In this case, the step count correcting apparatus 103 calculates the pre-correction step count X=C2-C1 for the livestock animal for the time slot "2012/02/20, 1:00 to 2:00".

The step count correcting apparatus 103 identifies from the relay device ID of the relay device 102 that relayed the received measurement result information, the area in which the livestock animal A was pastured during the time slot "2012/02/20, 1:00 to 2:00". For example, the relay device ID of the relay device 102 that relayed the measurement result information received by the step count correcting apparatus 103 on "2012/02/20" at "2:00" is assumed to be "B2". In the relay device DB 202, the area ID "area J2" is correlated with the relay device ID "B2".

Therefore, the step count correcting apparatus 103 identifies that during the time slot "1:00 to 2:0" on "2012/02/20", livestock animal A was pastured in the area J2. In this case, the step count correcting apparatus 103 multiplies X calculated above by the average correction value t for the area J2, to calculate post-correction step count Y=X×t calculated to correct for the terrain. Here, X=C2-C1, and t=0.7. t=0.7 is a value defined in the area DB 203-1 as the average correction value for the area J2.

The step count correcting apparatus 103 multiplies Y calculated above, by the vegetation and plant correction value for the area J2 to calculate the post-correction step count Z=Yxs calculated to correct for the terrain. As described above, here, Y=X×t and s=1.10. s=1.10 is a value defined in the vegetation and plants DB 204, as the vegetation and plant correction value for the period "January to March" for the vegetation and plants P2.

The step count correcting apparatus 103 stores Z calculated above to the time slot field "1:00 to 2:00" for "2012/02/20" in the step count DB 205. As described above, in the example depicted in FIG. 10, in the time slot field "1:00 to 2:00" for "2012/02/20", N302 is stored. In this case, N302=Z=(C2-C1)×0.7×1.10.

The estrus detection flag field has an ON/OFF field that stores information indicating whether the estrus detection flag is ON or OFF. Here, as one example, when the ON/OFF field stores "1", the estrus detection flag is indicated to be ON. On the other hand, when the ON/OFF field stores "0", the estrus detection flag is indicated to be OFF. The estrus detection flag field further has a flag ON date/time field that stores information indicating when the estrus detection flag was set to ON.

For example, when in the step count DB 205, the step counts of the livestock animal, stored in the time slot fields for the two most recent time slots are respectively determined to be greater than or equal to a given threshold, the step count correcting apparatus 103 sets the estrus detection flag to ON. For example, in the step count correcting apparatus 103, different thresholds for each time slot, such as a threshold for the step count of the livestock animal A during the time slot "0:00 to 1:00" and a threshold for the step count of the livestock animal A during the time slot "1:00 to 2:00", are defined. Further, the thresholds may be defined according to the abnormal state that the worker W wants to find. The thresholds may be defined stepwise, such that when the step count of the livestock animal A is less than a threshold Th1, illness is determined; when the step count is the threshold Th1 or greater and less than a threshold Th2, a normal state is determined; and when the step count is greater than the threshold Th2, estrus is determined.

The step count DB 205 further has relay device ID fields. For example, a relay device ID field stores information indicating the relay device ID of the relay device 102 that relayed to the step count correcting apparatus 103, the measurement result information last transmitted by the communications device 101.

In the step count DB 205, the fields described above are set for each communications device ID, enabling the step count DB 205 to store step count information for each communications device ID. The step count correcting apparatus 103 stores to the time slot field in the step count DB 205 and corresponding to the communications device ID received together with the measurement result information, the step count of the livestock animal A having the communications device 101 of the received communications device ID. In the example depicted in FIG. 10, the step count information stored for the communications device 101 of the communications device ID "G01" is depicted.

### (Functional configuration example of step count correcting apparatus 103)

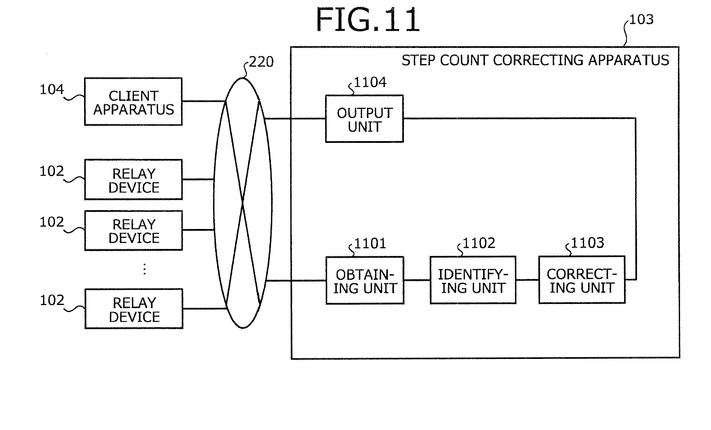

A functional configuration example of the step count correcting apparatus 103 will be described. FIG. 11 is a block diagram depicting a functional configuration example of the step count correcting apparatus 103. In FIG. 11, the step count correcting apparatus 103 includes an obtaining unit 1101, an identifying unit 1102, a correcting unit 1103, and an output unit 1104. These functions forming a control unit, e.g., the obtaining unit 1101 to the output unit 1104, for example, are implemented by executing on the CPU 501, a program stored on the magnetic disk 505 depicted in FIG. 5, or by the I/F 509 or the magnetic disk 505. Process results of the functional units are stored to the RAM 503, for example.

The obtaining unit 1101 has a function of obtaining at a specific interval, the measurement results of the step count of the livestock animal A and information of a specific relay device 102 used to report and the measurement results of the step count. For example, the obtaining unit 1101 receives, via the network 220, measurement result information, a communications device ID, and a relay device ID from a relay device 102, and thereby, obtains the measurement results of the step count of the livestock animal A and the information of the relay device 102 used to report the measurement results of the step count.

The identifying unit 1102 has a function of referring to correction values of the area correlated with the communications area of the specific relay device 102, based on the information of the specific relay device 102 obtained by the obtaining unit 1101, and identifying a specific area correction value that corresponds to the specific relay device 102. For example, the identifying unit 1102 identifies from the received relay device ID and the relay device DB 202, an area ID correlated with the same relay device ID as the received relay device ID. The identifying unit 1102 identifies from the identified area ID and the area DB 203-1, the average correction value correlated with the same area ID as the identified area ID.

Further, when the specific relay device 102 obtained by the obtaining unit 1101 has changed from the relay device 102 used for the previous report, the identifying unit 1102 may refer to an adjacent area correction value correlated with each set of communications areas of adjacent relay devices 102, to identify a specific adjacent area correction value correlated with the set of relay devices 102 including the specific relay device 102 and the relay device used for the previous report.

For example, from the currently received relay device ID and the relay device DB 202, the identifying unit 1102 identifies, as the migration destination area ID, the area ID of the area in which the relay device 102 of the currently received relay device ID is installed. From the relay device ID previously received and stored in the step count DB 205 and the relay device DB 202, the step count correcting apparatus 103 identifies, as the migration origin area ID, the area ID of the area in which the relay device 102 of the previously received relay device ID is installed. The identifying unit 1102 uses the identified migration origin area ID and migration destination area ID, and the adjacent area correction value DB 203-2 to identify an adjacent area correction value.

The identifying unit 1102, based on the information of the specific relay device 102 obtained by the obtaining unit 1101, may refer to a vegetation correction value correlated with the communications area of the relay device 102, to identify a specific vegetation correction value correlated with the specific relay device 102.

For example, the identifying unit 1102 identifies from the received relay device ID and the relay device DB 202, the area ID correlated with the same relay device ID as the received relay device ID. The identifying unit 1102 identifies from the identified area ID and the area DB 203-1, the vegetation and plant ID correlated with the same area ID as the identified area ID. The identifying unit 1102, from the identified vegetation and plant ID and the vegetation and plants DB 204, identifies the vegetation and plant correction value correlated with the same vegetation and plant ID as the identified vegetation and plant ID.

The correcting unit 1103 has a function of correcting the measurement results of the step count of the livestock animal A, based in the correction value identified by the identifying unit 1102. For example, the correcting unit 1103 multiplies the measurement results of the step count of the livestock animal A and the average correction value, to calculate the post-correction step count corrected for the terrain. If an adjacent area correction value is identified by the identifying unit 1102, the correcting unit 1103 multiplies the measurement results of the step count of the livestock animal A and the adjacent area correction value, to calculate the post-correction step count corrected for the terrain.

If a vegetation and plant correction value is identified by the identifying unit 1102, the correcting unit 1103 multiplies the measurement results of the step count of the livestock animal A and the vegetation and plant correction value, to calculate the post-correction step count corrected for the vegetation and plants. A concrete example of correction by the correcting unit 1103 will be described hereinafter.

The output unit 1104 has a function of outputting the measurement results of the step count of the livestock animal A after correction by the correcting unit 1103. For example, the output unit 1104, via the network 220, transmits to the client apparatus 104, the post-correction step count corrected by the correcting unit 1103.

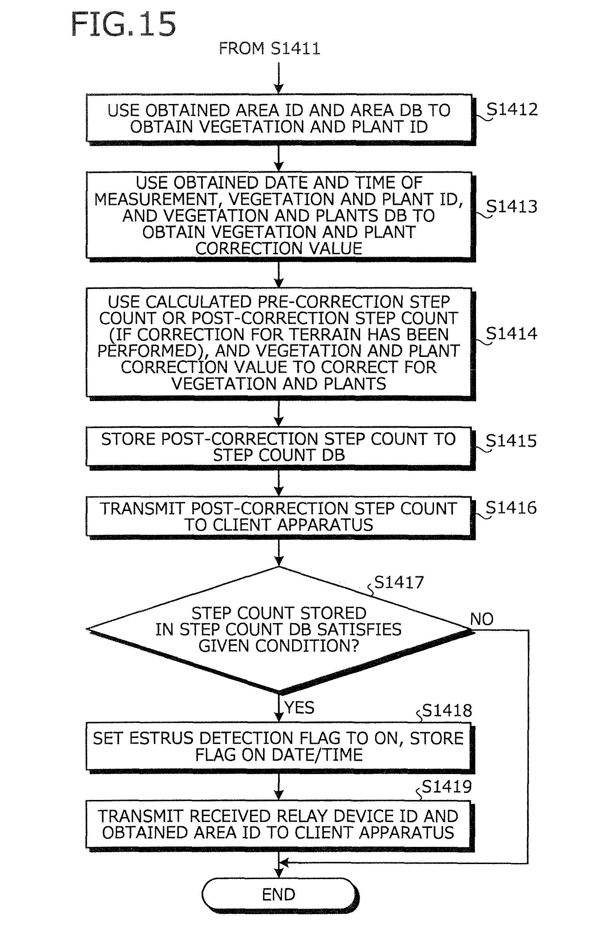

The step count correcting apparatus 103 further has a non-depicted determining unit, and may determine using the determining unit and based on the post-correction step count of the livestock animal A corrected by the correcting unit 1103, whether the livestock animal A is in estrus. Determination of estrus of the livestock animal A will be described with reference to FIG. 15. In this case, the output unit 1104 outputs identification information of the livestock animal A determined to be in estrus by the determining unit. For example, as the identification information of the livestock animal, the output unit 1104 transmits to the client apparatus 104, via the network 220, the communications device ID of the communications device 101 attached to the livestock animal A determined to be in estrus.

### (Example of display by client apparatus 104)

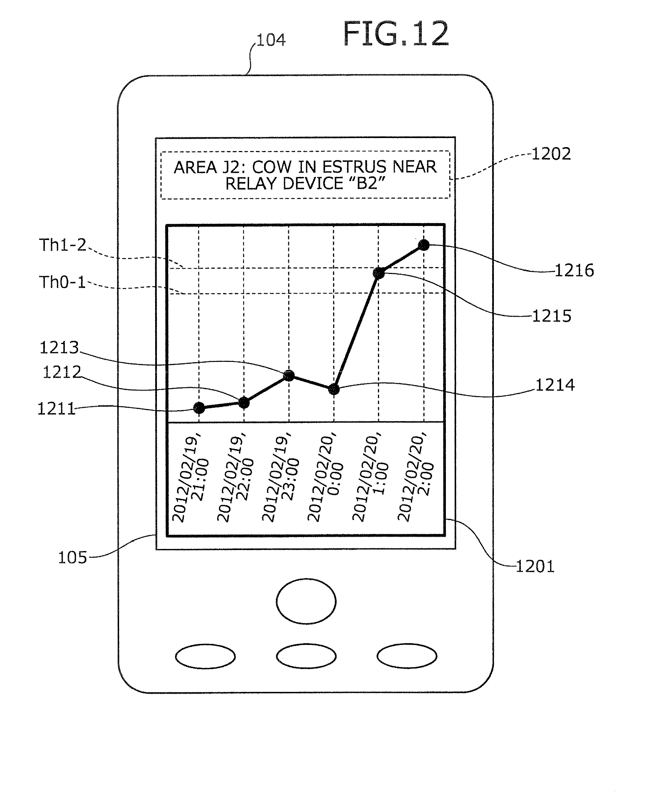

One example of display by the client apparatus 104 will be described. FIG. 12 is a diagram depicting one example of display by the client apparatus 104. As described above, the client apparatus 104 receives from the step count correcting apparatus 103, information indicating the step count of the livestock animal A. The information indicating the step count of the livestock animal A and received by the client apparatus 104 is information indicating the post-correction step count calculated by the step count correcting apparatus 103. The client apparatus 104, upon receiving the information indicating the step count from the step count correcting apparatus 103, stores the received information indicating the step count. The client apparatus 104 displays on the display 105, an image based on the stored information indicating the step count.

As depicted in FIG. 12, the client apparatus 104 displays a step count transition image 1201 representing transition of the step count of the livestock animal A over a given period. In the example depicted in FIG. 12, the client apparatus 104 displays the step count transition image 1201 representing the transition of the step count of the livestock animal A over a six-hour period from "21:00" on "2012/02/19" until "2:00" on "2012/02/20".

The step count correcting apparatus 103 receives measurement result information every hour, on the hour, calculates the post-correction step count, and transmits the post-correction step count to the client apparatus 104. The client apparatus 104 receives hourly from the step count correcting apparatus 103, information representing the step count of the livestock animal A and stores the received information.

In FIG. 12, a plot 1211 is a plot representing the step count of the livestock animal A received from the step count correcting apparatus 103 at "21:00" on "2012/02/19". A plot 1212 represents the step count of the livestock animal A received from the step count correcting apparatus 103 at "22:00" on the same date as the plot 1211. A plot 1213 represents the step count of the livestock animal A received from the step count correcting apparatus 103 at "23:00" on the same date as the plot 1211.

A plot 1214 represents the step count of the livestock animal A received from the step count correcting apparatus 103 at "0:00" on "2012/02/20". A plot 1215 represents the step count of the livestock animal A received from the step count correcting apparatus 103 at "1:00" on the same date as the plot 1214. A plot 1216 represents the step count of the livestock animal A received from the step count correcting apparatus 103 at "2:00" on the same date as the plot 1214. The client apparatus 104 displays on the display 105, the step count transition image 1201 that includes an image in which the plots 1211 to 1216 are connected by a line.

Here, for example, the step count of the livestock animal A represented by the plot 1215 is a value that is higher than a threshold Th0-1 set for the step count for the time slot from 0:00 to 1:00. Further, the step count of the livestock animal A represented by the plot 1216 is higher than a threshold Th1-2 set for the step count for the time slot from 1:00 to 2:00.

When the step counts of the two most recent time slots respectively are greater than or equal to a threshold, the step count correcting apparatus 103 notifies the client apparatus 104 that the livestock animal A has entered estrus. The client apparatus 104 receives the notification and displays on the display 105, a message 1202 pointing to the livestock animal A that has entered estrus. When giving notification of the livestock animal A in estrus, the step count correcting apparatus 103 may further transmit the relay device ID of the relay device 102 that relayed the measurement information transmitted by the communications device 101 attached to the livestock animal A. Upon receiving the relay device ID from the step count correcting apparatus 103, the client apparatus 104, by the message 1202, notifies the worker W of the received relay device ID as the relay device 102 near the livestock animal A.

### (Example of process performed by communications device 101)

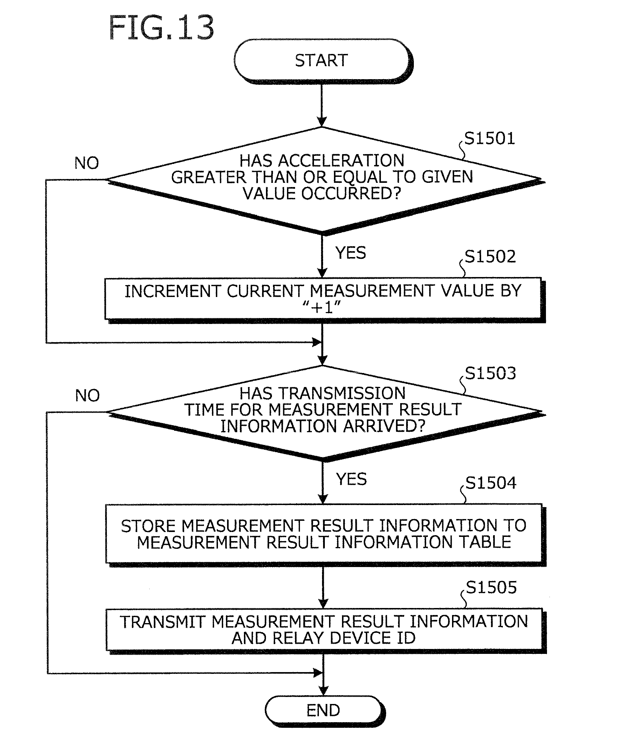

An example of a process performed by the communications device 101 will be described. FIG. 13 is a flowchart depicting an example of a process performed by the communications device 101. In FIG. 13, the communications device 101 determines from the output value of the sensor 304, whether acceleration of the communications device 101, greater than or equal to a given value has occurred (step S1301). If acceleration greater than or equal to the given value has not occurred (step S1301: NO), the communications device 101 transitions to the operation at step S1303.

If acceleration greater than or equal to the given value has occurred (step S1301: YES), the communications device 101 increments the current measurement value by "+1" (step S1302). Consequent to the operation at step S1302, the communications device 101 can cumulate the measurement value by incrementing the current measurement value by +1 each time the communications device 101 accelerates consequent to ambulation of the livestock animal A.

The communications device 101 determines from measurement results of the timer 305, whether the transmission time for the measurement result information has arrived (step S1303). For example, at step S1303, the communications device 101 determines that the transmission time for the measurement result information has arrived when the time measured by the timer 305 indicates each hour, on the hour. If the transmission time for the measurement result information has not arrived (step S1303: NO), the communications device 101 ends the series of operations according to the present flowchart.

If the transmission time for the measurement result information has arrived (step S1303: YES), the communications device 101 stores the measurement result information to the measurement result information table 201 (step S1304). For example, at step S1304, the communications device 101 stores to the measurement result information table 201, measurement result information in which the date and time at which the current measurement result information was transmitted and the current measurement value are correlated. Consequent to the operation at step S1304, the communications device 101 can store a measurement value each time measurement result information is transmitted.

The communications device 101 transmits to the relay device 102, each record of measurement result information stored in the measurement result information table 201 and the communications device ID thereof (step S1305), and ends the series of operations according to the present flowchart. Consequent to the operation at step S1305, the communications device 101 can transmit measurement result information at a given transmission interval. Further, consequent to the operation at step S1305, the communications device 101 can transmit not only the measurement value for the current transmission time of the measurement result information, but also the measurement values for past transmissions of the measurement result information.

As described above, the relay device 102, upon receiving the measurement result information and communications device ID transmitted from the communications device 101, transmits the received measurement result information and communications device ID, and the relay device ID thereof, to the step count correcting apparatus 103, via the network 220. Thus, the step count correcting apparatus 103 can obtain the measurement result information, the communications device ID, and the relay device ID, via the relay device 102.

The communications device 101 may reset the current measurement value to "0" at a given timing. For example, the communications device 101 sets the current measurement value to "0" at a given time, such as every hour, on the hour. The communications device 101 may set the current measurement value to "0", if an instruction to set the current measurement value to "0" is received from the step count correcting apparatus 103, via the relay device 102.

### (Example of process performed by step count correcting apparatus 103)

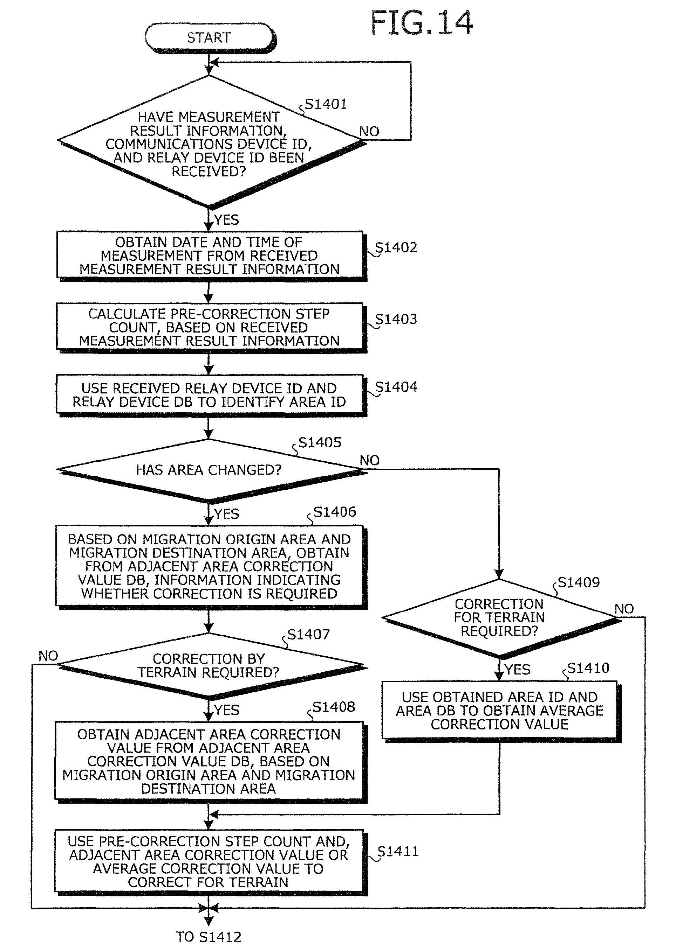

An example of a process performed by the step count correcting apparatus 103 will be described. FIG. 14 is a flowchart (part 1) depicting an example of a process performed by the step count correcting apparatus 103. FIG. 15 is a flowchart (part 2) depicting the example of the process performed by the step count correcting apparatus 103. As depicted in FIG. 14, the step count correcting apparatus 103 determines whether measurement result information, a communications device ID, and a relay device ID have been received (step S1401). The step count correcting apparatus 103 stands by until measurement result information, a communications device ID, and a relay device ID are received (step S1401: NO).

When measurement result information, a communications device ID, and a relay device ID have been received (step S1401: YES), the step count correcting apparatus 103 obtains the date and time of measurement from the received measurement result information (step S1402). The step count correcting apparatus 103 calculates a pre-correction step count of the livestock animal A for each time slot, based on the received measurement result information (step S1403).

The step count correcting apparatus 103 uses the relay device ID received at step S1401 and the relay device DB 202 to identify an area ID (step S1404). At step S1404, the step count correcting apparatus 103 obtains from the relay device DB 202, the area ID correlated with the same relay device ID as the received relay device ID. Consequent to the operation at step S1404, the step count correcting apparatus 103 can identify the area in which the livestock animal A is pastured.

The step count correcting apparatus 103 uses the area ID obtained at step S1404, the area DB 203-1, and the relay device ID stored in the step count DB 205 to determine whether the area in which the relay device 102 that relayed the current measurement result information is installed and the area in which the relay device 102 that relayed the previous measurement result information are different, indicating whether a target livestock animal A has moved to another area (step S1405).

At step S1405, if the areas are different (step S1405: YES), based on the migration origin area and the migration destination area, the step count correcting apparatus 103 obtains from the adjacent area correction value DB 203-2, information indicating whether correction is required (step S1406). At step S1405, if the areas are the same (step S1405: NO), the step count correcting apparatus 103 transitions to the operation at step S1409.

Based on the information obtained at step S1406 and indicating whether correction is required, the step count correcting apparatus 103 determines whether the terrain of the adjacent portion of the migration origin area and the migration destination area is terrain for which correction is required (step S1407). At step S1407, if correction is determined to be required (step S1407: YES), based on the area ID of the migration origin area and the area ID of the migration destination area, the step count correcting apparatus 103 obtains the adjacent area correction value from the adjacent area correction value DB 203-2 (step S1408), and transitions to the operation at step S1411. Further, at step S1407, if correction is determined to not be required (step S1407: NO), the step count correcting apparatus 103 transitions to the operation at step S1412.

At step S1409, the step count correcting apparatus 103 uses the area DB 203-1 to determine whether correction for the terrain is required for the area in which the target livestock animal A is located (step S1409). At step S1409, the step count correcting apparatus 103 determines whether, in the area DB 203-1, "correction by terrain" is "required" is stored for the area ID coinciding with the area ID obtained at step S1404. Consequent to the operation at step S1409, the step count correcting apparatus 103 can determine for each area, whether correction by the terrain is required. If correction by the terrain is not required (step S1409: NO), the step count correcting apparatus 103 transitions to the operation at step S1412 depicted in FIG. 15.

If correction by terrain is required (step S1409: YES), the step count correcting apparatus 103 uses the area ID obtained at step S1404 and the area DB 203-1 to obtain an average correction value (step S1410). At step S1410, the step count correcting apparatus 103 obtains the average correction value correlated with the same area ID as the obtained area ID. Consequent to the operation at step S1410, the step count correcting apparatus 103 can obtain the average correction value for each area. Concerning a livestock animal A for which it is known that the livestock animal A has changed areas, it is preferable to use the adjacent area correction value already obtained from the adjacent area correction value DB 203-2 until a given step count (e.g., 100 steps) is reached, and after the given step count is reached, to use an average correction value defined for each area.

The step count correcting apparatus 103 uses the pre-correction step count calculated at step S1403 and, the adjacent area correction value obtained at step S1406 or the average correction value obtained at step S1410 to correct for the terrain and calculate the post-correction step count (step S1411). At step S1411, the step count correcting apparatus 103 obtains, as the post-correction step corrected for the terrain, a value calculated by multiplying the pre-correction step count and, the adjacent area correction value or the average correction value. Consequent to the operation at step S1411, the step count correcting apparatus 103 can obtain the post-correction step count calculated by correcting for the terrain.

As depicted in FIG. 15, the step count correcting apparatus 103 uses the area ID obtained at step S1404 and the area DB 203-1 to obtain a vegetation and plant ID (step S1412). At step S1412, the step count correcting apparatus 103 obtains the vegetation and plant ID correlated with the same area ID as the obtained area ID. Consequent to the operation at step S1412, the step count correcting apparatus 103 can identify vegetation and plants for each area.

The step count correcting apparatus 103 uses the date and time of measurement obtained at step S1402, the vegetation and plant ID obtained at step S1412, and the vegetation and plants DB 204, to obtain a vegetation and plant correction value (step S1413). For example, at step S1413, the step count correcting apparatus 103 identifies the vegetation and plant correction value for each period, correlated with the same vegetation and plant ID as the obtained vegetation and plant ID. The step count correcting apparatus 103 obtains from among the identified vegetation and plant correction values for each period, the vegetation and plant correction value corresponding to the period in which the date and time of measurement are included. Consequent to the operation at step S1413, the step count correcting apparatus 103 can obtain a vegetation and plant correction value for each vegetation and plant and for each period.

The step count correcting apparatus 103 uses the pre-correction step count calculated at step S1403 or the post-correction step count calculated at step S1411, and the vegetation and plant correction value obtained at step S1413 to correct for the vegetation and plants. The step count correcting apparatus 103 calculates the post-correction step count (step S1414).

If correction for the terrain is performed at step S1411, at step S1414, the step count correcting apparatus 103 uses the post-correction step count corrected for the terrain. On the other hand, if correction for the terrain is not performed at step S1411, at step S1414, the step count correcting apparatus 103 uses the pre-correction step count calculated at step S1403. The step count correcting apparatus 103 obtains, as the post-correction step count corrected for the vegetation and plants, a value obtained by multiplying the pre-correction step count or the post-correction step count obtained by correcting for the terrain, and the vegetation and plant correction value. Consequent to the operation at step S1414, the step count correcting apparatus 103 can obtain the post-correction step count calculated by correcting for the vegetation and plants.

The step count correcting apparatus 103 stores as the step count of the livestock animal A and to the step count DB 205, the post-correction step count calculated at step S1414 (step S1415). At step S1415, in the step count DB 205, the step count correcting apparatus 103 stores the calculated post-correction step count into the time slot field identified by the received communications device ID and measurement result information. Consequent to the operation at step S1415, the step count correcting apparatus 103 can store the post-correction step count to the step count DB 205, as the step count of the livestock animal A. Further, when storing the step count of the livestock animal A, the step count correcting apparatus 103 stores into the relay device ID field of the step count DB 205, information representing the relay device ID received at step S1401.

The step count correcting apparatus 103 transmits information representing the post-correction step count calculated at step S1414, to the client apparatus 104 of the worker W (step S1416). Consequent to the operation at step S1416, the step count correcting apparatus 103 can transmit the post-correction step count to the client apparatus 104 and notify the worker W of the post-correction step count, as the step count of the livestock animal A.

The step count correcting apparatus 103 determines whether the step count of the livestock animal A stored in the step count DB 205 satisfies a given condition (step S1417). For example, at step S1417, the step count correcting apparatus 103 determines whether among the step counts of the livestock animal A stored in the step count DB 205, the step counts of the two most recent time slots are respectively greater than or equal to a threshold. Without limitation hereto, the given threshold can be arbitrarily determined according to the abnormal state of the livestock animal A the worker W desires to detect. Consequent to the operation at step S1417, the step count correcting apparatus 103 can determine whether an abnormal state has occurred with the livestock animal A.

If the step counts do not satisfy the given threshold (step S1417: NO), the step count correcting apparatus 103 ends the series of operations according to the present flowchart. If the step counts of the livestock animal A stored in the step count DB 205 satisfy the given condition (step S1417: YES), the step count correcting apparatus 103 sets the estrus detection flag in the step count DB 205 to ON (step S1418). When setting the estrus detection flag to ON, the step count correcting apparatus 103 stores the flag ON date/time. Consequent to the operation at step S1414, the step count correcting apparatus 103 can store the detection of an abnormal state of the livestock animal A and the date and time of the detection.

The step count correcting apparatus 103 transmits the relay device ID received at step S1401 and the area ID obtained at step S1404 to the client apparatus 104 of the worker W (step S1419), and ends the series of operations according to the present flowchart. Consequent to the operation at step S1419, the step count correcting apparatus 103 can store detection of an abnormal state of the livestock animal A and notify the worker W of the relay device 102 set near the livestock animal A and/or the area in which the livestock animal A is pastured. From the contents notified by the step count correcting apparatus 103, the worker W can surmise the location of the livestock animal A for which an abnormal state has occurred and can narrow down the range to be searched for the livestock animal A. Therefore, the step count correcting apparatus 103 can reduce the work load of the worker W when searching for the livestock animal A.

As described, the step count correcting apparatus 103 of the present embodiment corrects for the terrain of an area, enabling the measured step count of the livestock animal A to be converted into a step count for a standardized terrain. As a result, the step count correcting apparatus 103 can reduce variations in the step count of the livestock animal A consequent to the effects of the terrain. Therefore, the step count correcting apparatus 103 can suppress variations in the step count consequent to the effects of vegetation and plants and improve the accuracy when detecting from the step counts of the livestock animals A, a livestock animal A in an abnormal state.

The step count correcting apparatus 103 of the present embodiment corrects for the vegetation and plants of the area, enabling the calculated the step count of the livestock animal A to be converted into a step count for a standardized terrain. Thus, the step count correcting apparatus 103 can reduce variation of the step count of the livestock animal A consequent to the effects of the vegetation and plants. As a result, the step count correcting apparatus 103 can suppress variations in the step count consequent to the effects of vegetation and plants and improve the accuracy when detecting from the step counts of the livestock animals A, a livestock animal A in an abnormal state.

The step count correcting apparatus 103 stores vegetation and plant correction values for the vegetation and plants, respectively, and can correct for the vegetation and plants growing, according to the period. Thus, the step count correcting apparatus 103 can further reduce variation of the step count of the livestock animal consequent to the effects of vegetation and plants, enabling the accuracy to be further improved when detecting from the step counts of the livestock animals A, a livestock animal A in an abnormal state.

In the present embodiment, although flat terrain is set as a reference; an average correction value of a value less than "1.0" is set for terrain having slopes; and the step count of the livestock animal A measured in terrain having slopes is converted to a step count for flat terrain, configuration is not limited hereto. For example, on a farm F, a greater part of which is occupied by an area having slopes, the step count of the livestock animal A measured in flat terrain may be multiplied by an average correction value of a value greater than "1.0".

Further, although a technique of attaching a communications apparatus equipped with a GPS function to the livestock animal A may be considered, attaching a communications apparatus equipped with a GPS function to each livestock animal A has a high cost. Furthermore, the GPS function continuously emits an electronic signal and therefore, the consumption of the battery, which is driving source, is fast, requiring frequent replacement of the battery or the communications apparatus itself. Consequently, this technique not only has a high initial cost but also places a burden on the worker W in terms of battery or apparatus replacement.

On the contrary, the step count correcting apparatus 103 identifies the area in which the livestock animal A is pastured, based on the relay device ID of the relay device 102 that relayed the measurement information transmitted from the communications device 101 and therefore, can reduce the initial cost as well as the work load of the worker W, for battery replacement.

In the present embodiment, although an example is described in which average correction values are stored in the area DB 203-1 and vegetation and plant correction values are stored in the vegetation and plants DB 204, configuration is not limited hereto. For example, in the area DB 203-1, for each area ID, both an average correction value and a vegetation and plant correction value may be stored. Further, for each area ID, correction values enabling corrective effects by both meteorological correction values and soil correction value of the present embodiment to be obtained may be stored in a correction value DB. In this case, the step count correcting apparatus 103 identifies in the correction value DB, one correction value from the area ID and multiplies the pre-correction step count by the identified correction value, enabling correction for the terrain and correction for the vegetation and plants to be performed by one calculation.

The step count correcting method described in the present embodiment may be implemented by executing a prepared program on a computer such as a personal computer and a workstation. The program is stored on a computer-readable recording medium such as a hard disk, a flexible disk, a CD-ROM, an MO, and a DVD, read out from the computer-readable medium, and executed by the computer. The program may be distributed through a network such as the Internet.

### EXPLANATIONS OF LETTERS OR NUMERALS

- 101: communications device
- 102: relay device
- 103: step count correcting apparatus
- 104: client apparatus

## Claims

1. A step count correcting method executed by a computer that corrects a step count measurement result received, via a relay device, from a step counting device attached to a livestock animal, the step count correcting method comprising:
obtaining at a specific interval, the step count measurement result of the livestock animal and information of a specific relay device used to report the step count measurement result;
identifying a specific area correction value that corresponds to the specific relay device, by referring to the relay device and an area correction value correlated with a communications area of the relay device;
correcting the step count measurement result based on the specific area correction value; and
outputting the corrected step count measurement result.

2. The step count correcting method according to claim 1, wherein
the identifying includes referring to an adjacent area correction value correlated with each set of communications areas of adjacent relay devices, when the specific relay device used to report has changed from the relay device used to report previously, and identifying a specific adjacent area correction value that corresponds to a set including the specific relay device and the relay device used to report previously, and
the correcting includes correcting the step count measurement result based on the specific area correction value and the specific adjacent area correction value.

3. The step count correcting method according to claim 1 or 2, wherein
the identifying includes referring to the relay device and a vegetation correction value correlated with vegetation of the communications area of the relay device, and identifying a specific vegetation correction value that corresponds to the specific relay device, and
the correcting includes correcting the step count measurement result based on the area correction value and the specific vegetation correction value.

4. The step count correcting method according to claim 1, wherein
the identifying includes referring to the relay device and a period-specific vegetation correction value correlated with a period and vegetation of the communications area of the relay device, and identifying a specific period-specific vegetation correction value that corresponds to a period when the specific relay device and the step count measurement result are obtained, and
the correcting includes correcting the step count measurement result based on the area correction value and the specific period-specific vegetation correction value.

5. A step count correcting method executed by a computer that determines estrus, based on a step count measurement result from a step counting device attached to a livestock animal, the step count correcting method comprising:
obtaining the step count measurement result of the livestock animal and information of a specific relay device used to report the step count measurement result;
identifying a specific area correction value that corresponds to the specific relay device, by referring to a correspondence relation of the relay device and an area correction value that corresponds to a communications area of the relay device;
determining estrus of the livestock animal, based on the specific area correction value and the step count measurement result; and
outputting identification information of the livestock animal determined to be in estrus.

6. A step count correcting apparatus that corrects a step count measurement result received, via a relay device, from a step counting device attached to a livestock animal, the step count correcting apparatus comprising:
an obtaining unit that obtains at a specific interval, the step count measurement result of the livestock animal and information of a specific relay device used to report the step count measurement result;
an identifying unit that identifies a specific area correction value that corresponds to the specific relay device, by referring to the relay device and an area correction value correlated with a communications area of the relay device; and
a correcting unit that corrects the step count measurement result based on the specific area correction value; and
an output unit that outputs the step count measurement result corrected by the correcting unit.

7. The step count correcting apparatus according to claim 6, wherein
the identifying unit refers to an adjacent area correction value correlated with each set of communications areas of adjacent relay devices, when the specific relay device used to report has changed from the relay device used to report previously, and identifies a specific adjacent area correction value that corresponds to a set including the specific relay device and the relay device used to report previously, and
the correcting unit corrects the step count measurement result based on the specific area correction value and the specific adjacent area correction value.

8. The step count correcting apparatus according to claim 6 or 7, wherein
the identifying unit refers to the relay device and a vegetation correction value correlated with vegetation of the communications area of the relay device, and identifies a specific vegetation correction value that corresponds to the specific relay device, and
the correcting unit corrects the step count measurement result based on the area correction value and the specific vegetation correction value.

9. The step count correcting apparatus according to claim 6, wherein
the identifying unit refers to the relay device and a period-specific vegetation correction value correlated with a period and vegetation of the communications area of the relay device, and identifies a specific period-specific vegetation correction value that corresponds to a period when the specific relay device and the step count measurement result are obtained, and
the correcting unit corrects the step count measurement result based on the area correction value and the specific period-specific vegetation correction value.

10. A step count correcting apparatus that determines estrus, based on a step count measurement result from a step counting device attached to a livestock animal, the step count correcting apparatus comprising:
an obtaining unit that obtains the step count measurement result of the livestock animal and information of a specific relay device used to report the step count measurement result;
an identifying unit that identifies a specific area correction value that corresponds to the specific relay device, by referring to a correspondence relation of the relay device and an area correction value that corresponds to a communications area of the relay device;
a determining unit that determines estrus of the livestock animal, based on the specific area correction value and the step count measurement result; and
an output unit that outputs identification information of the livestock animal determined to be in estrus by the determining unit.
